(19) Europäisches Patentamt

European Patent Office

Office européen des brevets

(11) **EP 0 604 387 B1**

(12) **EUROPÄISCHE PATENTSCHRIFT**

(45) Veröffentlichungstag und Bekanntmachung des Hinweises auf die Patenterteilung:
**26.06.1996 Patentblatt 1996/26**

(51) Int Cl.[6]: **G12B 17/06**, G01K 1/02, A61L 2/24, A61L 2/26, B01J 3/04

(21) Anmeldenummer: **93890244.2**

(22) Anmeldetag: **15.12.1993**

(54) **Messanordnung**

Measuring arrangement

Installation de mesure

(84) Benannte Vertragsstaaten:
**AT BE CH DE DK ES FR GB IT LI NL SE**

(30) Priorität: **15.12.1992 AT 2481/92**

(43) Veröffentlichungstag der Anmeldung:
**29.06.1994 Patentblatt 1994/26**

(73) Patentinhaber: **SBM SCHOELLER-BLECKMANN Medizintechnik Gesellschaft m.b.H.**
**A-2630 Ternitz (AT)**

(72) Erfinder:
• **Binder, Hans**
**A-1200 Wien (AT)**
• **Lohninger, Gerd**
**A-2630 Ternitz (AT)**

(74) Vertreter: **Weinzinger, Arnulf, Dipl.-Ing. et al Patentanwälte**
**Sonn, Pawloy, Weinzinger & Wolfram**
**Riemergasse 14**
**A-1010 Wien (AT)**

(56) Entgegenhaltungen:
**EP-A- 0 168 518**     **FR-A- 2 219 405**
**GB-A- 1 152 833**     **GB-A- 2 151 030**
**US-A- 4 059 764**     **US-A- 4 800 513**

## Beschreibung

Die Erfindung betrifft eine Meßanordnung für Einrichtungen mit erschwerten klimatischen Bedingungen, z.3. Autoklaven, mit mindestens einem Meßfühler zur Erfassung von physikalischen Meßgrößen, z.B. einem Temperaturmeßfühler, mit einer mit dem Meßfühler über eine Verbindungsleitung verbundenen bzw. verbindbaren Meßelektronik, die in einem thermisch isolierten Behälter angeordnet ist und eine Sendeeinheit enthält, der eine außerhalb des Behälters an einem Deckel hiefür angeordnete, über eine Verbindungsleitung angeschlossene bzw. anschließbare Sendeantenne zugeordnet ist, und mit einer gesonderten Empfängereinheit, der eine Empfangsantenne zugeordnet ist.

Für die Meßtechnik ergeben sich des öfteren Situationen, wo unter schwierigen bzw. kritischen Bedingungen, wie etwa in Dampf- Atmosphären, Heißwassersterilisatoren, oder ganz allgemein bei hohen Temperaturen, hohen Drücken und dergl., bestimmte Meßgrößen, wie Temperatur, Druck usw., zu erfassen sind. Ein ganz typisches Beispiel hiefür sind die Dampfsterilisatoren oder Sterilisations-Autoklaven, wie sie beispielsweise in der Lebensmittelindustrie oder pharmazeutischen Industrie verwendet werden, um Lebensmittel bzw. Infusionen, Lösungen und dergl. Produkte bei erhöhter Temperatur und erhöhtem Druck mittels Wasserdampf zu sterilisieren. Dabei ist in der Regel zur Validierung und Prozeßsteuerung des Sterilisationsprozesses vor allem in der pharmazeutischen Industrie eine hochgenaue Temperaturmessung an mehreren Stellen im Autoklaven erforderlich. Hiefür werden bei stationären Autoklaven üblicherweise Temperaturfühler mittels Durchführungen in den Autoklaven eingebracht. Eine derartige Technik ist jedoch bei umlaufenden Autoklaven, den sog. Rotationsautoklaven, nicht möglich. Herkömmliche Apparaturen für Rotationsautoklaven speichern daher Meßwerte während der Sterilisationsbehandlung, und sie können erst nach erfolgter Sterilisation Auskunft über deren Verlauf geben, nicht jedoch Meßwerte während des Behandlungsprozesses übertragen, etwa um ein rasches Reagieren bei sich ändernden Sterilisationsbedingungen zu ermöglichen.

Eine derartige Meßwertspeicherung in einem elektronischen Speicher im Inneren eines temperaturisolierten Behälters ist beispielsweise in Zusammenhang mit der Sterilisation von Konserven in der DE-C-23 08 887 beschrieben, wobei im einzelnen auf eine möglichst störungsfreie Meßelektronik abgestellt ist. Eine solche Speicherung von Meßwerten, insbesondere Temperaturmeßwerten, während eines Behandlungsvorganges mag zwar bei der Sterilisation von Konservendosen akzeptabel sein, da hier die Sterilisationsbehandlung nicht so besonders kritisch ist und daher nur selten, wenn eine nachfolgende Auswertung der gespeicherten Meßwerte dies ergibt, eine Wiederholung der Sterilisationsbehandlung erforderlich ist; anders verhält es sich jedoch bei den vorstehend genannten pharmazeutischen Produkten, wo eine Sterilisationstemperatur mit großer Genauigkeit über einen exakten Zeitraum eingehalten werden muß, um die Sterilisation im gewünschten Ausmaß sicherzustellen. Wenn dort erst im nachhinein auf Basis der gespeicherten Meßwerte eine Abweichung der Sterilisationstemperatur während eines bestimmten Zeitraums festgestellt wird, muß für diese Produkte der Sterilisationsvorgang wiederholt werden, so daß sich in der Praxis aufgrund zahlreicher derartiger Behandlungswiederholungen ein hoher Zeitaufwand - und damit Kostenaufwand - ergibt. In der Praxis wird deshalb aus Sicherheitsgründen zumeist von vornherein generell eine überlange Sterilisationsdauer vorgesehen, so daß sich Behandlungswiederholungen nur selten als notwendig erweisen; jedoch wird dies mit dem Nachteil erkauft, daß ganz allgemein eine vergleichsweise lange Sterilisationszeit erforderlich ist.

Andererseits ist das Problem einer ausreichenden thermischen Isolierung bei derartigen abgeschlossenen Meßwertspeicherbehältern leichter lösbar als in jenen Fällen, wo laufend eine Meßwertübertragung nach außen verlangt ist. Dies ist nicht nur darauf zurückzuführen, daß die Anschlüsse für die Meßwertfühler dicht auszuführen sind, sondern auch darauf, daß die Elektronik im thermisch isolierten Behälter einfacher gehalten werden kann, wodurch in der Folge auch vergleichsweise wenig Platz benötigt wird. Dies führt dazu, daß bei vorgegebenen Außenabmessungen des Behälters auch bei einer sehr aufwendigen Isolierung die Meßelektronik samt Speicher und Batterie noch genügend Platz im Inneren des Behälters findet. Wenn jedoch, im Hinblick auf die Meßwertübertragung, der Schaltungsaufwand erhöht werden muß, erhöht sich damit einhergehend auch der Platzbedarf im Inneren des Behälters, wodurch bei vorgegebenen Außenabmessungen des Behälters die thermische Isolierung schwächer ausgelegt werden muß. Eine Vergrößerung des Behälters ist andererseits zumeist nicht möglich, da in den Beschickungswagen für Autoklaven normalerweise nur beschränkt Platz für derartige Behälter vorgesehen ist. Abgesehen davon erhöht sich bekanntlich mit Vergrößerung der Außenoberfläche eines Behälters - aufgrund der damit vergrößerten Wärmedurchgangsfläche - der Isolierungsbedarf, wodurch erst bei relativ weitreichenden Behältervergrößerungen ein merkbarer Raumgewinn möglich ist.

Besondere Temperaturschutzgefäße für Meßeinrichtungen, die jedoch aufgrund von eingebauten Wassergefäßen konstruktiv sehr aufwendig sind, ergeben sich weiters aus der DE-C-25 09 787 sowie der DE-A-27 20 118. Ferner ist in der DE-C-35 45 215 ebenfalls ein Temperaturschutzgefäß für eine batteriebetriebene Meßwertspeicherschaltung beschrieben, wobei dort als vorteilhaft herausgestellt wird, daß sich aufgrund der Speicherung der Meßwerte in der elektronischen Speicherschaltung die ansonsten notwendige Schleppkabelverbindung zu einer Anzeigestation und eine hochfrequente Signalübertragung aus dem Ofen während der Ofenreise des Durchlaufofenwagens erübrigen kann. Wie dargelegt, bringt zwar eine derartige Meßwertspeicherung eine bauliche bzw. schaltungsmäßige Vereinfachung mit sich, hat jedoch bei kritischeren Produkten den Nachteil einer häufigen Wiederholung von Wärmebehandlungen, insbesondere Sterilisationsbehandlungen im Fall von pharmazeutischen Produkten.

Zu erwähnen ist auch, daß aus der US-A-4 059 764 ein Infrarotsensor mit mehreren Sensorelementen in einem Array bekannt ist, wobei diese Sensorelementanordnung auf einer Leiterplatine im Hohlraum eines einfachen Dewar-Gefäßes und zwar in dessen Bodenbereich, angebracht ist, und die Infrarotstrahlung durch ein IR-durchlässiges Fenster des Dewar-Gefäßes eintreten kann. Die Sensorelemente sind mit äußeren Anschlußstiften bzw. Anschlußleitungen über Leitungen unter Durchführung durch die Dewar-Gefäß-Wand verbunden und können so an die übrige - nicht näher erläuterte - Meßeinrichtung angeschlossen werden.

In Zusammenhang mit Dampfsterilisatoren wurde ferner in der AT-B-388 502 bereits ganz allgemein eine Meßdatenübertragung aus dem Sterilisator per Funk vorgeschlagen; als Temperaturschutzbehälter wird ein metallischer Doppelmantelbehälter vorgeschlagen, wobei der Hohlraum zwischen den Mänteln evakuiert und mit Kieselgur gefüllt sein soll. Die Meßelektronik mit der Senderschaltung ist im Inneren dieses Behälters in Paraffin eingegossen, und sie ist über elektrisch isolierende Durchleitungen im Deckel des metallischen Behälters mit den einzelnen Meßsonden verbunden. Tatsächlich hat sich jedoch diese bekannte Meßanordnung in der Praxis nie durchgesetzt, und mit ähnlichen Meßanordungen durchgeführte Versuche, die zur vorliegenden Erfindung geführt haben, haben gezeigt, daß mit derartigen Meßanordnungen nur eine ungenügende Funktion erreicht werden kann. Im einzelnen ergeben sich insbesondere eine ungenügende thermische Isolierung der Meßelektronik im metallischen Behälter und in der Folge eine fehlerhafte Meßwertübertragung, abgesehen davon, daß auch die Leitungsdurchführung im Deckel des Behälters nicht ausreichend dicht auszuführen und umständlich in der Handhabung ist; insgesamt ergeben sich daher bei der bekannten Meßanordnung äußerst kurze Betriebszeiten, d.h. die bekannte Meßanordnung kann jeweils nur für einen eher kurzen Sterilisationsvorgang eingesetzt werden, danach muß der Behälter gegen einen anderen getauscht werden, da im zuvor verwendeten Behälter bereits eine zu hohe Innentemperatur herrscht, abgesehen davon, daß die Schaltung nach relativ kurzer Zeit wieder ein Nachladen der verwendeten Batterien erforderlich macht. Diese Nachteile haben offensichtlich dazu geführt, daß in der Praxis bei Dampfsterilisatoren nach wie vor Meßanordnungen mit Meßwertspeicherung, wie weiter oben erläutert, verwendet werden.

Andererseits ist es auch bekannt, zur Überwachung der Temperatur von rotierenden Maschinenteilen, wie etwa Heizwalzen oder Rotoren von elektrischen Maschinen, die Temperaturmeßdaten drahtlos zu einer stationäre Einrichtung zu übertragen, wobei hiefür insbesondere der Einsatz von Licht emittierenden Dioden (LEDs), in Zusammenhang mit einer modulierten Lichtstrahlung, vgl. z.B. EP-B-75 620 sowie DE-A-28 52 679, bzw. eine kapazitive Übertragung mittels eines Kondensators vorgesehen wird, von dem eine Platte mit dem rotierenden Teil der Maschine mitrotiert und die Gegenplatte stationär angeordnet ist, vgl. z.B. DE-C-24 28 890. Derartige Übertragungseinrichtungen zur drahtlosen Meßwertübertragung sind jedoch ganz offensichtlich bei Dampfsterilisatoren bzw. Autoklaven nicht bzw. nicht ohne weiteres anwendbar, vor allem weil z.B. durch den vorhandenen Dampf die Lichtübertragung mit Hilfe von LEDs beeinträchtigt würde, abgesehen davon, daß bei den hohen Temperaturen die LEDs kaum ausreichend thermisch isoliert werden könnten; in ähnlicher Weise würde die kapazitive Übertragung durch den zwischen den Kondensatorplatten vorhandenen Dampf verfälscht werden.

Es ist daher ein Ziel der Erfindung, eine Meßanordnung wie oben angegebenen zu schaffen, mit der eine störungsfreie drahtlose Meßwertübermittlung über lange Zykluszeiten möglich ist, so daß eine sichere und wirtschaftliche Messung z.B. der Temperatur auch in rotierenden Autoklaven möglich ist; dabei wird auch ein möglichst geringer baulicher Aufwand angestrebt, und eine exakte laufende Messung und Auswertung der Meßergebnisse, ohne Verfälschung durch Temperatureinflüsse oder dergl., soll ferner On-line-Eingriffe beim mit der Meßanordnung überwachten Prozeß, z.B. Sterilisationsprozeß, im zuverlässiger Weise ermöglichen.

Zur Lösung dieser Aufgabe ist die Meßanordnung der eingangs angeführten Art dadurch gekennzeichnet, daß der thermisch isolierte Behälter zwei zueinander entgegengesetzt ausgerichtete, ineinander angeordnete Dewar-Gefäße enthält, von denen das innere Dewar-Gefäß die Meßelektronik aufnimmt, wobei die Verbindungsleitungen im Zwischenraum zwischen den Dewar-Gefäßen hindurchgeführt und an im Deckel dicht angebrachten Kupplungsteilen angeschlossen sind, denen korrespondierende Kupplungsteile zum Anschließen der Sendeantenne bzw. der Verbindungsleitung zum wenigstens einen Meßfühler zugeordnet sind, und daß die Meßelektronik einen Analog/Digital-Konverter zur Digitalisierung der Meßdaten sowie einen digitalen Steuer- und Prozessorbaustein enthält, dem die Sendeeinheit nachgeschaltet ist.

Bei der vorliegenden Meßanordnung ist eine funktionssichere drahtlose Meßwertübermittlung von analogen Meßgrößen, wie Druck, Temperatur etc., in rotierenden - oder aber auch in statischen - Autoklaven oder dergl. über längere Zeiträume oder Zykluszeiten möglich, wobei mit dem angegebenen thermisch isolierten Behälter (der sog. "Meßflasche"), der im Autoklaven untergebracht wird, einerseits eine gute thermische Isolierung und andererseits ausreichend Platz für die darin enthaltende elektronische Komponenten sowie für zur Stromversorgung vorgesehene aufladbare Batterien (Akkumulatoren) sichergestellt wird.

Mehr im einzelnen ist dabei von wesentlicher Bedeutung, daß daß die beiden ineinander angeordneten Dewar-Gefäße, die relativ eng ineinander sitzen, etwa mit einem Zwischenraum von 0,5 mm, die angestrebte ausreichende thermische Isolierung ermöglichen, wobei überraschenderweise auch die Leitungsführung im Zwischenraum zwischen den Dewar-Gefäßen die Isolierungswirkung nicht beeinträchtigt und eine übermäßige Erwärmung der Meßelektronik im Inneren der Dewar-Gefäße sicher vermieden werden kann. Es ergibt sich nämlich für eine etwaige Wärmeübertragung ein relativ langer Weg zwischen den beiden ineinandergesteckten Dewar-Gefäßen, der der Leitungsführung für die Verbindungsleitungen zwischen der Meßelektronik und den Kupplungsteilen am Deckel entspricht. Dieser lange Weg reicht überraschend aus, um das Entstehen nachteiliger Wärmebrücken von der Umgebung zur Meßelektronik zu verhindern. Für die gewünschte Isolierung sowie überdies für den Zusammenbau des Behälters mit den ineinander angeordneten Dewar-Gefäßen sind weiters die im Deckel angebrachten Kupplungsteile von Bedeutung, die nicht nur den gewünschten dichten Abschluß problemlos sicherstellen, im Gegensatz zu einfachen Kabeldurchführungen, sondern überdies auch die (Vor-)Montage der Meßflasche, mit der innenliegenden Meßelektronik, erleichtern. Im Hinblick auf das bei vorgegebenen Außenabmessungen der Meßflasche durch die ineinander angeordneten Dewar-Gefäße verminderte Platzangebot, das die Verwendung von verhältnismäßig kleinen aufladbaren Batterien bzw. Akkus erforderlich macht, ist ein Ausführen der Meßelektronik mit geringem Stromverbrauch notwendig, um zu akzeptablen Zykluszeiten zu kommen, und dies wird mit der angegebenen Meßdaten-Digitalisierung und den dafür einsetzbaren digitalen Schaltungskomponenten sichergestellt.

In der Folge wird eine hohe Meßgenauigkeit auch bei hohen Autoklaventemperaturen erzielt, und es wird eine laufende Überwachung der gemessenen Größen, insbesondere Temperatur, ermöglicht. Bei Sterilisationsbehandlungen für die pharmazeutische Industrie besteht dann ferner z.B. die Möglichkeit der Ermittlung des sog. F0-Wertes (der F0-Wert ist eine übliche Kenngröße für ein bestimmtes Sterilisationsausmaß) und damit der Optimierung des Sterilisationsprozesses in On-Line-Technik, wobei es möglich ist, die Sterilisation bei Erreichen eines vorgegebenen F0-Wertes abzubrechen, wodurch Zeit, Energie und Kosten gespart werden können.

Um die Meßelektronik immer erst unmittelbar vor ihrem Einsatz im Autoklaven bequem einschalten und sofort nach dem Einsatz zwecks Stromeinsparung wieder ausschalten zu können, sowie um die Batterie einfach wieder aufladen zu können, ist es erfindungsgemäß von besonderem Vorteil, wenn im Deckel des thermisch isolierten Behälters weiters ein mit einer aufladbaren Batterie bzw. mit Stromversorgungsklemmen der Meßelektronik verbundener Kupplungsteil angebracht ist. Es liegen somit für alle Anschlüsse, für jene zu den Meßfühlern ebenso wie für die Sendeantenne und auch für die Batterieaufladung bzw. die Aus/Ein- Schaltung, vergleichbare, dicht angeführte Kupplungen im Deckel des thermisch isolierten Behälters vor. Bei diesen elektrischen Kupplungen kann es sich im Prinzip beispielsweise um Rohrverschraubungen handeln, vorteilhafterweise sind jedoch die Kupplungsteile Steckverbindungsteile. Die Kupplungsteile bzw. Steckverbindungsteile, die im Deckel angebracht sind, können dabei relativ einfach, etwa mit O-Ringen oder dergl., dicht und wärmedämmend montiert werden, und bei Verwendung des thermisch isolierten Behälters mit der Meßelektronik können sodann die außen anzuschließenden Komponenten, nämlich Meßfühler bzw. Sendeantenne, je nach Bedarf einfach angesteckt bzw. eingeschraubt werden.

Für eine besonders gute thermische Isolierung hat es sich weiters als zweckmäßig erwiesen, wenn die beiden Dewar-Gefäße in einer Isolierschicht aus Aluminiumoxidkeramik eingebettet sind.

Im Hinblick auf die erfindungsgemäß vorgesehene Digitalisierung der Meßdaten in der im thermisch isolierten Behälter enthaltenen Meßelektronik und der damit einhergehenden stromsparenden Auslegung der digitalen Schaltung ist es auch möglich, den erfindungsgemäß vorgesehenen Prozessor- und Steuer-baustein ohne erhöhten Stromverbrauch zusätzlich für weitere Funktionen heranzuziehen, und demgemäß ist es von besonderem Vorteil, wenn der Prozessor- und Steuerbaustein einen Codierer zur Codierung der digitalisierten Meßdaten, z.B. einen Hamming-Codierer, enthält. Auf diese Weise kann ohne zusätzlichen hohen Aufwand zusätzlich eine Fehlerkorrektur bei der Meßdatenermittlung und -übertragung vorgesehen werden, wobei im einzelnen beispielsweise 1-Bit-Fehler korrigiert und Mehrbit-Fehler erkannt werden können. Einzelne Datenpakete mit einem Mehrbit-Fehler können dann z.B. verworfen werden, und es wird erst das nächste Datenpaket, entsprechend der nächsten Messung, ausgewertet, vorausgesetzt, daß dann kein Mehrbit-Fehler enthalten ist.

Aus Stromeinsparungsgründen ist es weiters besonders vorteilhaft, wenn der Prozessor- und Steuerbaustein die Sendeeinheit abwechselnd ein- und ausschaltet. Dabei kann die Ruhezeit beispielsweise 3 bis 3,5 s und die Ein- oder Sendezeit z.B. 0,6 s betragen. Auch ist es möglich, bei starken Änderungen der Meßgrößen kürzere, insbesondere variable Aus-Ein-Schaltzyklen (etwa mit einem Tastverhältnis von 2:1 statt wie oben angegeben ca. 5:1 bis 6:1) vorzusehen. Auf diese Weise benötigt die Sendeeinheit nur während eines Bruchteils der Zeit eine Stromversorgung, was sich gerade in diesem Schaltungsteil mit eher hohem Stromverbrauch besonders günstig auswirkt.

Auch ist es von Vorteil, wenn zur Überwachung der Temperatur im Inneren der Dewar-Gefäße, im Bereich der Meßelektronik, ein interner Temperaturmeßfühler angeordnet ist. Bei einer derartigen Ausbildung kann aufgrund der überwachten Innentemperatur nicht nur auf eine zu hohe Innentemperatur hin zum Schutz der Elektronik sofort eingegriffen werden, sondern auch, falls erforderlich, eine temperaturabhängige Meßwertkorrektur an den übertragenen Meßwertdaten vorgenommen werden.

Zwecks Schaltungsvereinfachung, um mit möglichst wenig Platz im Inneren der Dewar-Gefäße des thermisch isolierten Behälters das Auslagen zu finden, ist es ferner gerade im vorliegenden Zusammenhang besonders günstig, wenn im Fall von mehreren Meßfühlern diesen in an sich bekannter Weise eine Multiplexereinheit, für einen Multiplex-Betrieb der Meßfühler, zugeordnet ist.

Um temperaturabhängige Schwankungen bei der analogen Meßsignal-Verstärkung möglichst einfach berücksichtigen zu können, ohne die im thermisch isolierten Behälter enthaltene Meßelektronik besonders aufwendig gestalten zu müssen, ist es ferner günstig, wenn die Meßelektronik eine dem Analog/Digital-Konverter vorgeschaltete Überwachungsschaltung für Gleichspannungsparameter, wie insbesondere Gain und Offset, eines eingangsseitigen, analogen Meßsignal-Verstärkers der Meßelektronik enthält, und von der Überwachungsschaltung abgegebene, auf diese Gleichspannungsparameter bezogene Überwachungssignale nach Digitalisierung im Analog/Digital-Konverter an den Prozessor- und Steuer-Baustein zur Übermittlung an den Empfänger angelegt werden. Dabei ist eine besonders einfache, schaltungstechnisch effiziente Lösung erzielbar, wenn die Überwachungsschaltung mit einer Meßschaltung mit Referenzwiderständen ausgebildet ist, wobei im Betrieb der jeweilige Spannungsabfall am Meßwiderstand zwecks Bestimmung von Gain bzw. Offset gemessen wird.

Zum Senden der Meßdaten können an sich die verschiedensten für sich bekannten Modulationstechniken, wie insbesondere PCM etc., verwendet werden. Um im Hinblick auf das geringe Platzangebot die Schaltung möglichst einfach und klein ausführen zu können, ist es jedoch besonders vorteilhaft, wenn der Prozessor- und Steuerbaustein mit einer einen Eingangsteil der Sendeeinheit bildenden Frequenzverschiebungsschaltung verbunden ist, die bei Anlegen von Datenimpulsen durch den Prozessor- und Steuerbaustein eine Verschiebung der Sendefrequenz der Sendeeinheit bewirkt.

Insbesondere in einem Rotationsautoklaven, bei dem ein Autoklav-Rotor mit Hilfe von Rollen in einem Autoklav-Stator drehbar gelagert ist, werden an sich die über Funk übertragenen, die Meßdaten enthaltenden Funksignale relativ stark gestört, was nicht nur auf die Dampfatmosphäre im Inneren des Autoklaven zurückzuführen ist, sondern auch auf die Abschirmungswirkung des Autoklav-Rotors, die besonders störend ist, wenn sich beispielsweise die Sendeantenne auf der von einer etwa stabförmigen Empfangsantenne abgelegenen Seite befindet. Um hier Abhilfe zu schaffen und eine sichere Funksignalübertragung bzw. einen störungsfreien Funksignalempfang sicherzustellen, hat es sich auch als günstig erwiesen, wenn die Empfangsantenne durch einen zur Anbringung längs der Innenfläche einer äußeren Autoklaven-Wand vorgesehene, eine geschlossene, allgemein kreisförmige Schleife bildende Rahmenantenne gebildet ist. Bei dieser Rahmenantenne in Form einer Kreisschleife handelt es sich somit um eine sog. Cubical-Quad-Antenne, welche an zwei einander diametral gegenüberliegenden Stellen mit dem Autoklav-Stator elektrisch verbunden wird, und von der das HF-Signal in einem Abstand von einem Verbindungspunkt zum Stator, beispielsweise gleich ungefähr 60 cm, ausgekoppelt wird. Die wirksame Länge der Rahmenantenne hängt von der verwendeten Funkfrequenz ab, wobei sich eine Länge von ungefähr 11 m, was den ungefähren Innenabmessungen eines üblichen Autoklav-Stators entspricht, bei einer Sendefrequenz von ca. 27 MHz ergibt.

Von Vorteil ist es sodann, wenn in der Empfängereinheit ein Digitalisierungsbaustein sowie ein Prozessorsystem, gegebenenfalls mit einem dem Codierer des Prozessor- und Steuerbausteins der Meßelektronik im thermisch isolierten Behälter entsprechenden Decoder, vorgesehen ist, um die aktuellen Meßdaten, wie Fühlertemperaturen, aus den durch Funk übertragenen Signalen zu gewinnen. Dabei kann das Prozessorsystem im gegebenen Fall auch eine Korrektur der Meßdaten im Hinblick auf etwaige Schwankungen der Gleichspannungsparameter des analogen Verstärkers der Meßelektronik in der Meßflasche vornehmen. Diese Meßwertkorrektur kann durch einfaches Multiplizieren der gemessenen, übertragenen Werte, z.B. für die Temperatur, mit dem auf einen Referenzwert bezogenen Gain-Wert und durch eine Verschiebung entsprechend dem Offset-Wert vorgenommen werden, d.h. es gilt beispielsweise:

$$T_{(ist)} = [T_{(gemessen)} \cdot \text{Gain/Referenz}] + \text{Offset}$$

Für eine On-Line-Überwachung des Sterilisationsprozesses mit sofortiger Eingriffsmöglichkeit, etwa wenn die Sterilisationstemperatur um einen vorherbestimmten Pegel absinkt, ist es weiters günstig, an das Prozessorsystem des Empfängers einen externen Rechner, z.B. einen sog. Personal Computer (PC), anzuschließen, um diesem die Meßdaten, gegebenenfalls nach Meßwertwie vorstehend angegeben, für eine On-Line-Auswertung zu übermitteln. Dabei kann weiters im Rechner mit Vorteil eine Kennlinienkalibrierung für jeden Teil-Meßbereich, abhängig von vorhergehenden Eichungen der Meßfühler, durchgeführt werden.

Die Erfindung wird nachstehend anhand von in der Zeichnung veranschaulichten, besonders bevorzugten Ausführungsbeispielen, auf die sie jedoch nicht beschränkt sein soll, noch weiter erläutert. Im einzelnen zeigen:

Fig. 1 eine schematische Querschnittsdarstellung eines Sterilisations-Rotationsautoklaven mit einer erfindungsgemäßen Temperatur-Meßanordnung;

Fig. 2 eine schematische Axialschnittdarstellung eines solchen Rotationsautoklaven;

Fig. 3 eine in einem solchen Rotationsautoklaven unterzubringende sog. Meßflasche (thermisch isolierter Behälter) der erfindungsgemäßen Meßanordnung in einem axialen Schnitt;

Fig. 4 eine schematische Stirnansicht des inneren Dewar-Gefäßes (von dessen offener Seite her) mit den darin enthaltenen Meßelektronik-Komponenten einschließlich vier aufladbarer Batterien (Akkus);

Fig. 5 eine Draufsicht auf den Deckel der Meßflasche, zur Veranschaulichung der Löcher für die dicht anzubringenden Steckverbindungsteile;

die Fig. 6 und 7 in einer Draufsicht bzw. Ansicht einen Steckverbindungsteil (Buchsenteil) zur Anbringung im Deckel der Meßflasche;

Fig. 8 eine Ansicht eines komplementären Steckverbindungsteils oder Steckers, der mit einem Steckverbindungsteil (Buchsenteil) gemäß Fig. 6 und 7 zusammenarbeitet;

Fig. 9 ein Blockschaltbild der in der Meßflasche enthaltenen Meßelektronik der Meßanordnung;

Fig. 10 ein detaillierteres Schaltbild eines analogen Meßsignal-Verstärkers und einer Multiplexereinheit dieser Meßelektronik gemäß Fig. 9;

Fig. 11 ein detaillierteres Schaltbild einer Frequenzverschiebungsschaltung der Sendeeinheit der Meßelektronik gemäß Fig. 9;

Fig. 12 eine Ansicht einer dieser Sendeeinheit zugeordneten Sendeantenne mit Steckverbindungsteil;

Fig. 13 eine als Empfangsantenne verwendete Rahmenantenne in einer schematischen Stirnansicht, wobei auch die Wand des Autoklav-Stators schematisch dargestellt ist;

Fig. 14 ein Blockschaltbild der externen Empfängereinheit; und

Fig. 15 ein Flußdiagramm zur Veranschaulichung der in der Empfängereinheit durchgeführten Meßwertkorrektur bei sich ändernden Offset- und Gain-Werten des analogen Meßsignal-Verstärkers der Meßelektronik gemäß Fig. 9 und 10.

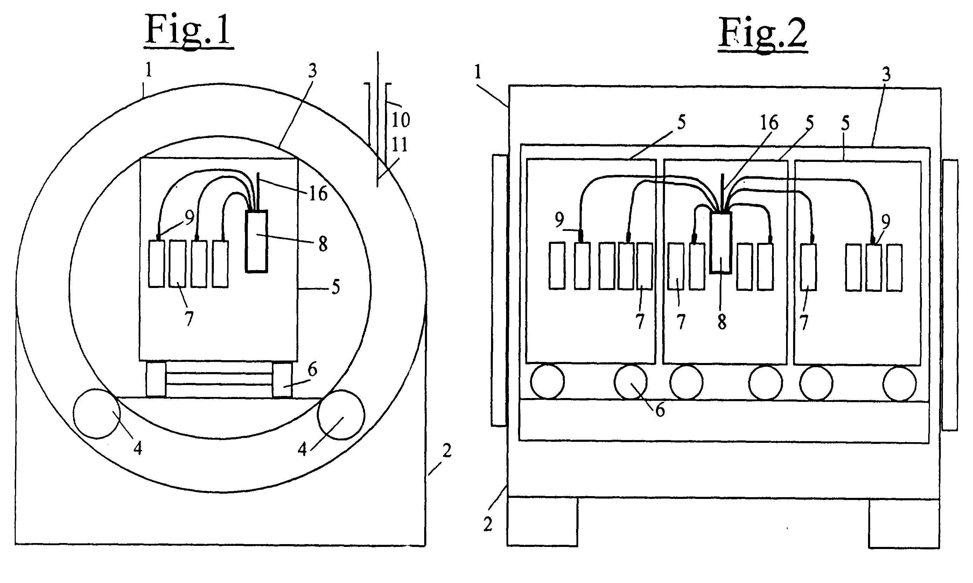

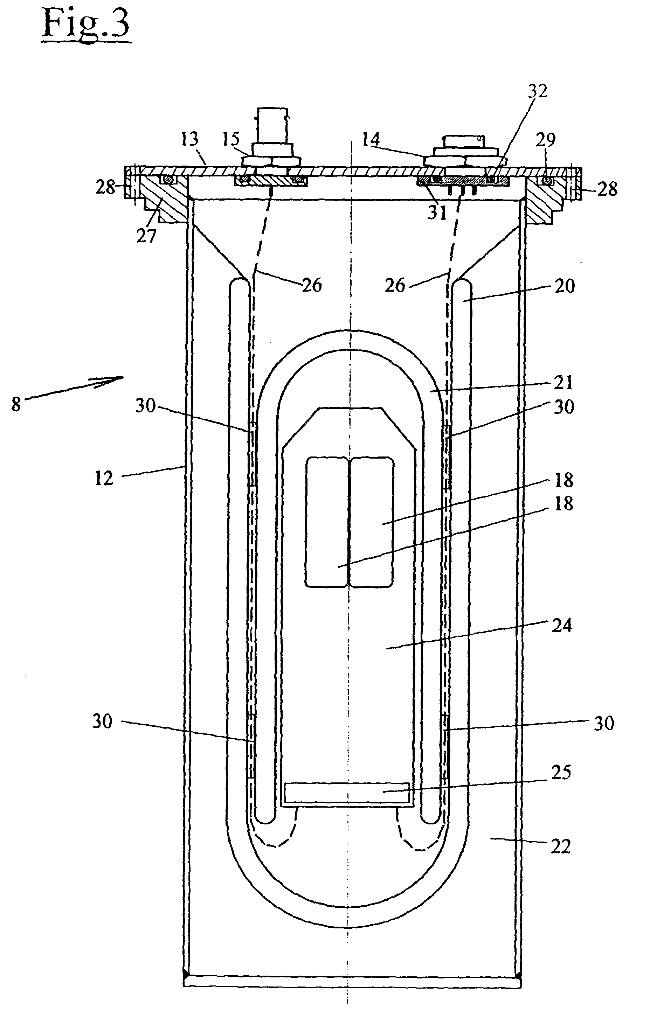

In Fig. 1 und 2 ist ein an sich herkömmlicher, allgemein mit 1 bezeichneter Rotationsautoklav veranschaulicht, bei dem die vorliegende Meßanordnung mit besonderem Vorteil eingesetzt werden kann, und der einen feststehenden Teil, den Autoklav-Stator 2, und einen rotierenden Teil, den Autoklav-Rotor 3, enthält. Dabei ist der Autoklav-Rotor 3 über Rollen 4 (s. Fig. 1) im Autoklav- Stator 2 drehbar gelagert; diese Rollen 4 sind in mehreren Paaren über die axiale Länge des Autoklav-Rotors 3 verteilt angeordnet und stützen dabei den käfigartigen Autoklav-Rotor 3 über nicht näher veranschaulichte Laufringe desselben ab, wie dies an sich bekannt ist und hier keiner weiteren Erläuterung bedarf. Zur Rotation des Autoklav-Rotors 3 wird zumindest eine der Rollen 4 drehend angetrieben.

In den Autoklav-Rotor 3 werden bei der durchzuführenden Sterilisationsbehandlung gesonderte Beschickungswagen 5 in axialer Richtung eingefahren, wozu sie mit entsprechenden, aus Fig. 2 ersichtlichen Laufrollen 6 ausgestattet sind. Die Beschickungswagen 5 enthalten das Sterilisationsgut, z.B. Glasflaschen 7 mit pharmazeutischen Produkten.

Die diesem Rotationsautoklaven zugeordnete Meßanordnung weist gemäß Fig. 1 und 2 einen Meßsender mit einem thermisch isolierten Behälter, eine sog. Meßflasche 8 auf, mit der Meßfühler 9, beispielsweise Temperaturfühler, verbunden sind. Die Meßflasche 8 bzw. die Meßfühler 9 sind dabei gemäß Fig. 1 und 2 den verschiedenen Beschickungswagen 5 des Rotationsautoklaven 1 bzw. den darin angeordneten Glasflaschen 7 zugeordnet.

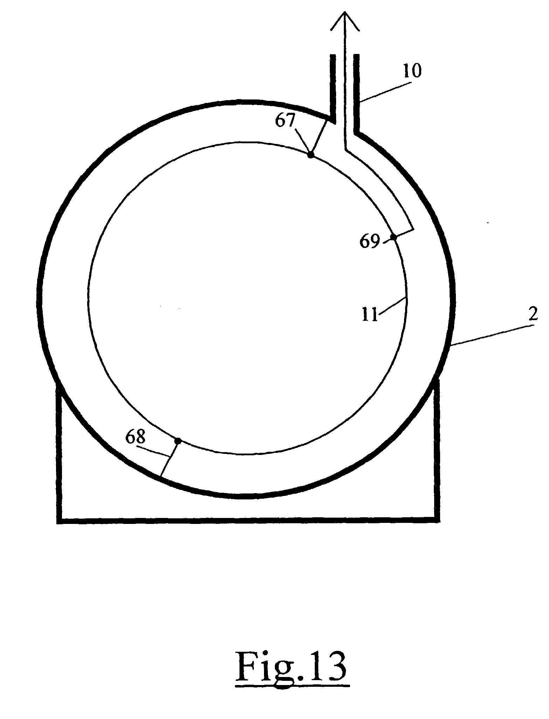

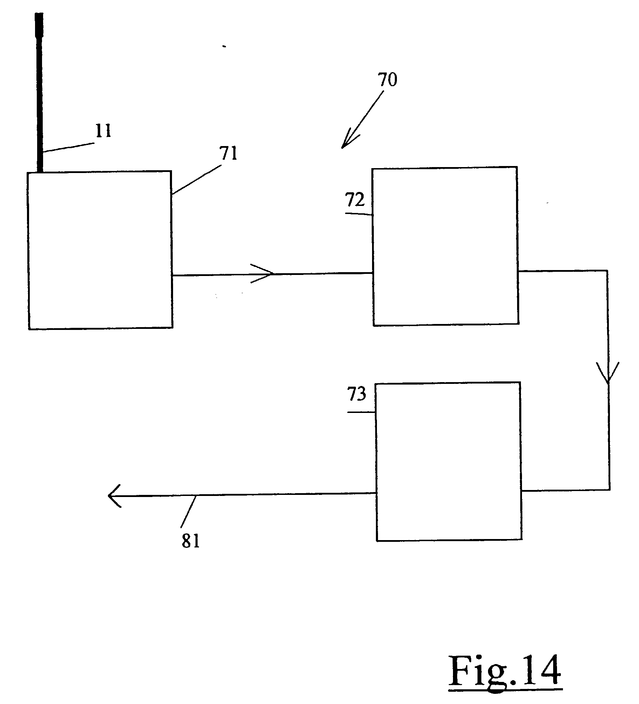

In Fig.1 ist noch eine am Autoklav-Stator 2 über eine Durchführung 10 angebrachte, einer nachstehend noch näher - anhand der Fig. 14 - zu erläuternden Empfängereinheit zugeordnete Empfangsantenne 11 ganz schematisch veranschaulicht, wobei im folgenden anhand von Fig. 13 eine besonders bevorzugte Ausführungsform der Empfangsantenne mehr im Detail erläutert werden soll.

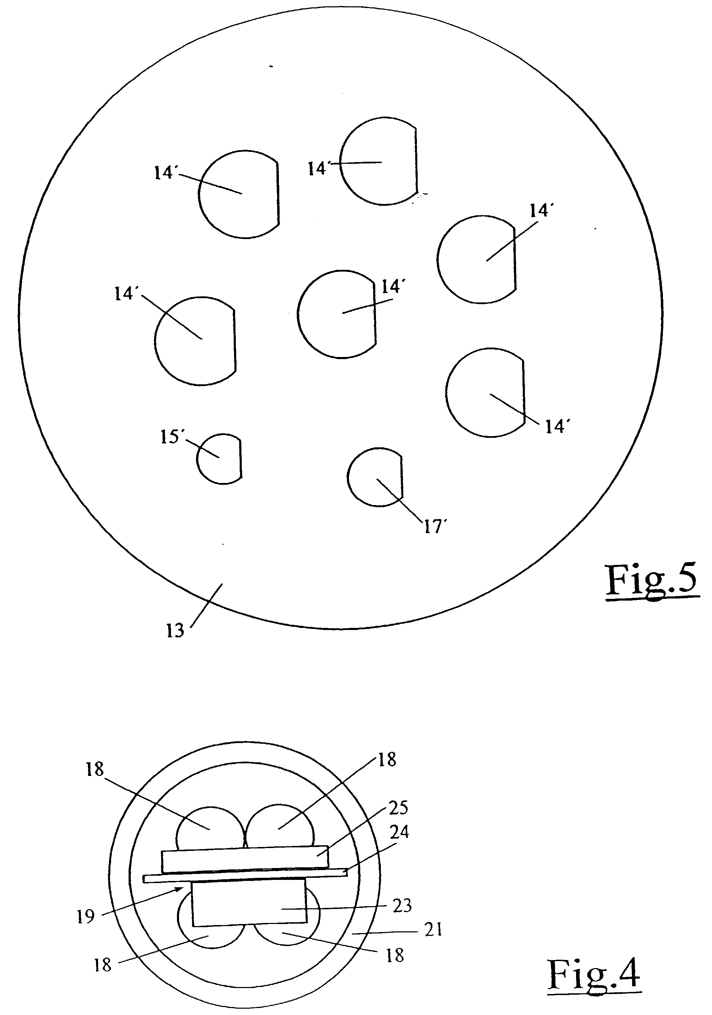

In Fig. 3 bis 5 ist die Meßflasche 8 mehr im einzelnen veranschaulicht, wobei diese Meßflasche 8 im wesentlichen aus einer Edelstahlflasche als Stahlmantel 12 mit einem Deckel 13 mit z.B. sechs (s. Fig. 5) hermetisch abgedichteten Steckverbindungsteilen 14, für insgesamt sechs Temperatur-Meßfühler 9, einem eine längere HF-Buchse aufweisenden Steckverbindungsteil 15 für eine Sendeantenne 16 (s. Fig. 1, 2, 9 und 12) und einem Steckverbindungsteil 17 für einen Lade- bzw. Verbindungsstecker für im Inneren der Meßflasche 8 enthaltene aufladbare Batterien 18 bzw. für deren Verbindung mit einer in der Meßflasche 8 enthaltenen Meßelektronik 19 besteht. Es sei erwähnt, daß in Fig. 5 nur die z.B. mittels eines Laserstrahls in den aus Stahl bestehenden Deckel 13 geschnittenen Löcher für die jeweiligen Steckverbindungsteile gezeigt sind, wobei die Löcher mit den Bezugszahlen der Steckverbindungsteile 14, 15 bzw. 17 entsprechenden Bezugszahlen, versehen mit einem Apostroph, also mit 14', 15' bzw. 17', bezeichnet sind. Weiters sei erwähnt, daß Fig. 5 - im Gegensatz zu Fig. 3 - auch ein zentrales Loch 14' für einen zentralen Steckverbindungsteil 14 veranschaulicht.

Die insgesamt vier Batterien 18, die auch aus Fig. 4 hinsichtlich ihrer Zahl und Anordnung ersichtlich sich, dienen zur Stromversorgung der Meßelektronik 19, und die Einheit, bestehend aus Batterien 18 und Elektronik 19, befindet sich dabei im Inneren von zwei eng (z.B. mit einem Abstand von 0,5 mm) ineinanderpassenden Glasdewar-Gefäßen 20, 21, von denen das innere Gefäß 21 mit der Öffnung nach unten und das äußere Gefäß 20 mit der Öffnung nach oben weist. Zur zusätzlichen thermischen Isolierung dient eine Füllung 22 aus Aluoxidkeramik. Im übrigen wird die Meßflasche 8 so gut wie möglich evakuiert.

Wie weiters aus Fig. 3 und 4 ersichtlich ist, wird die Meßelektronik 19 einschließlich einer Sendeeinheit 23 an einer Leiterplatine 24 angebracht, die auch einen mehrpoligen Stecker 25 für die verschiedenen Verbindungsleitungen 26 zu den Steckverbindungsteilen 14, 15 und 17 am Deckel 13 trägt.

Aus Fig. 3 ist auch ersichtlich, daß der Deckel 13 der Meßflasche 8 über einen am Stahlmantel 12 oben angeschweißten Flanschring 27 angeschraubt wird, wie schematisch bei 28 angedeutet ist, wobei überdies ein O-Ring 29 zwecks dichtem Abschluß zwischengelegt ist. Es sei erwähnt, daß in der schematischen Draufsicht von Fig. 5 die über den Umfang des Deckels 13 verteilt angeordneten Bolzenlöcher für die Schraubverbindung 28 der Einfachheit halber weggelassen wurden.

Ferner ist in Fig. 3 schematisch bei 30 veranschaulicht, daß die beiden Dewar-Gefäße 20, 21 mit Hilfe von verteilt angeordneten Haftschichten aneinander fixiert sind, wobei eine einheitliche Distanz - beispielsweise die oben erwähnten 0,5 mm - zwischen den beiden Dewar-Gefäßen 20 und 21 festgelegt wird. In diesem schmalen Zwischenraum zwischen den beiden Dewar-Gefäßen 20, 21 verlaufen wie erwähnt die Verbindungsleitungen 26 vom Stecker 25 an der Leiterplatine 24 zu den am Deckel 13 angebrachten Steckverbindungsteilen 14, 15 und 17, wobei durch die gezeigte Anordnung ein relativ langer Weg für diese Verbindungsleitungen 26 gegeben ist, der überraschenderweise ausreicht, um die Bildung von Wärmebrücken vom Deckel 13 bzw. von den darin angebrachten Steckverbindungsteilen 14, 15, 17 zur Meßelektronik 19 im Inneren des inneren Dewar-Gefäßes 21 zu verhindern. Im übrigen wird ein Wärmeübergang von der Umgebung der Meßflasche 8 zu Meßelektronik 19 auch durch das Vakuum im Inneren der Meßflasche 8 hintangehalten. Vor allem aber sehen die beiden Dewar-Gefäße 20, 21 eine ganz wesentliche Isolierungswirkung vor.

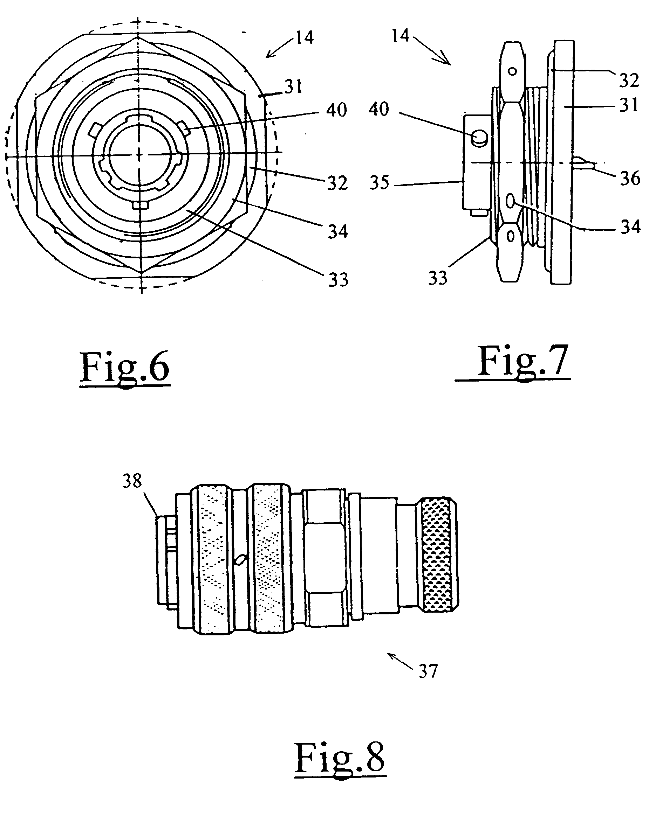

In Fig. 6 und 7 sind in einer Draufsicht bzw. Seitenansicht Steckverbindungsteile von an sich herkömmlicher Bauart der Vollständigkeit halber veranschaulicht, wobei diese Steckverbindungsteile beispielsweise jene sind, die zum Anschließen der Verbindungsleitungen zu den Meßfühlern 2 dienen und vorstehend mit 14 bezeichnet wurden. Es sei erwähnt. daß die Steckverbindungsteile 15 bzw. 17 für die Sendeantenne 16 bzw. für die Nachladung der Batterien 18 sowie das Anschließen der Meßelektronik 19 an die Batterien 18 im Prinzip gleich, wenn auch mit etwas unterschiedlichen Abmessungen, ausgeführt sein können.

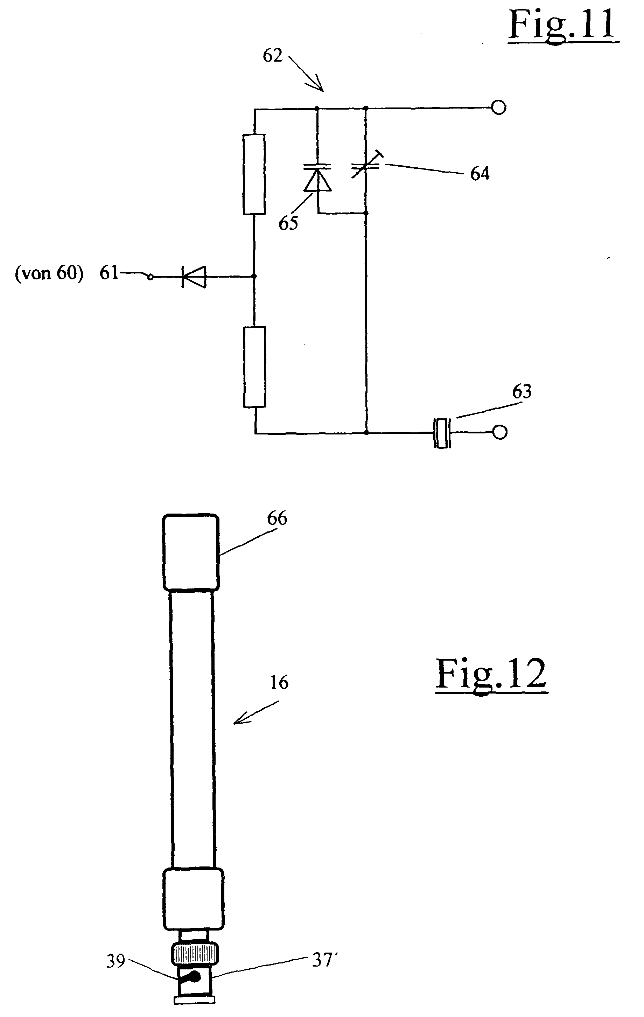

Die am Deckel 13 angebrachten Steckverbindungsteile, z.B. 14, weisen dabei in an sich herkömmlicher Weise eine untere Anschlagplatte 31 mit einem darin eingelassenen O-Ring 32 auf, der in der montierten Stellung (vgl. auch Fig. 3) an der Unterseite des Deckels 13 zu liegen kommt. Auf einen einteilig mit der unteren Anschlagplatte 31 verbundenen Gewindestutzen 33 ist ein Befestigungsring 34 aufgeschraubt. Als eigentlicher elektrischer Verbindungsteil dient eine Anschlußbuchse 35, die z.B. einen (oder mehrere) mit einer (oder mehreren) Anschlußfahne(n) 36 an der Unterseite des Steckverbindungsteiles 14 in elektrischer Verbindung stehende(n) Kontakt(e) (nicht gezeigt) enthält und mit einem entsprechenden, komplementären elektrischen Steckerteil 37 (s. Fig. 8) zusammenarbeitet, der mit der Verbindungsleitung zum jeweiligen Meßfühler 9 verbunden ist bzw. an der Sendeantenne 16 (vgl. Fig. 12) bzw. an einem nicht näher veranschaulichten Anschlußkabel für die Batterienachladung bzw. einem Verbindungsbauteil für das Anschalten der Meßelektronik 19 an die Batterien 18 angebracht ist.

Mit einem dicht am Deckel 13 befestigten Steckverbindungsteil 14 (bzw. 15 oder 17) wie vorstehend beschrieben kann beispielsweise das Eindringen von Luft in das Innere der evakuierten Meßflasche 8 auf einen Wert von $1 \times 10^{-7} cm^3$ pro Sekunde beschränkt werden.

In Fig. 8 ist beispielsweise eine ebenfalls an sich herkömmliche Bauart für den Steckerteil 37 in einer Ansicht gezeigt, wobei dieser Steckerteil 37 beispielsweise zum Anschließen der Verbindungsleitungen für die Meßfühler 9 über einen auf der rechten Seite des Steckerteils 37 gemäß Fig. 8 aufzusetzenden, nicht gezeigten, herkömmlichen Schrumpfadapter ausgebildet ist. Auf der gemäß Fig. 8 linken Seite weist der Steckerteil 37 einen zum Buchsenteil 35 des Steckverbindungsteils 14 gemäß Fig. 6 und 7 komplementären Kupplungsteil 38 auf, wobei in an sich herkömmlicher Weise eine Bajonettverbindung mit in Fig. 8 nicht näher ersichtlichen Schlitzen (vgl. jedoch auch Fig. 12, wo an einem vergleichbaren HF-Steckerteil 37' ein derartiger Schlitz bei 39 schematisch veranschaulicht ist) vorgesehen ist, in die die aus Fig. 6 und 7 ersichtlichen radialen Stifte 40 eingeführt werden.

Derartige Kupplungen oder Steckverbindungen, wie vorstehend anhand der Fig. 6 bis 8 erläutert, sind wie erwähnt an sich bekannt, so daß sich eine weitere Erläuterung erübrigen kann.

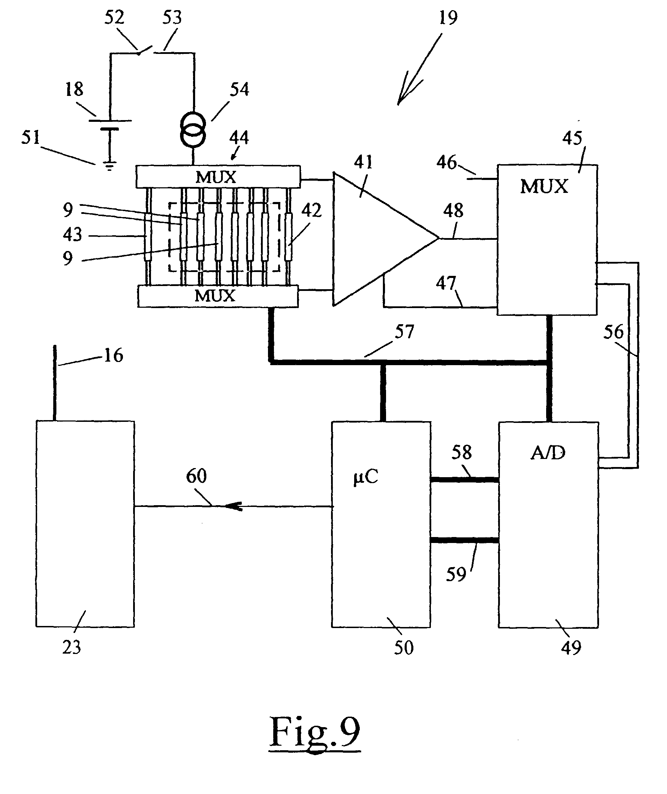

Gemäß Fig.9 besteht die Meßflaschen-Elektronik 15 allgemein aus einem analogen Meßsignal-Präzisionsverstärker 41, der mit den Meßfühlern 9 - z.B. Pt 100-Temperaturmeßwiderständen - sowie - in diesem Beispiel - mit einem in der Meßflasche 8 angebrachten internen Temperaturmeßfühler 42, etwa einem Innentemperaturmeßwiderstand, und ferner einem Referenz-Meßwiderstand 43 verbunden ist, weiters aus einer der Gruppe von Meßwiderständen bzw. Fühlern, 9, 42 und 43 zugeordneten Multiplexer-Einheit 44, ferner aus einer Multiplexer-Überwachungsschaltung 45 zur Messung der Batteriespannung (Anschluß 46) sowie insbesondere zur Überwachung von Gleichspannungsparametern des Verstärkers 41, nämlich Offset und Gain (Leitung 47), und zur Aufnahme und Weiterleitung der eigentlichen Meßsignale, die von den Meßfühlern 2, 42 herrühren (Leitung 48), im Multiplexbetrieb, weiters aus einem A/D-Konverter 49, aus einem Steuer- und Prozessorbaustein in Form eines Microcontrollers (μC) 50 und aus der Sendeeinheit (HF- Teil) 23 mit der daran angeschlossenen Sendeantenne 16. Die Multiplexereinheit 44 ist zur Umschaltung der Meßfühler bzw. -widerstände 9, 42, 43 vorgesehen, und ein entsprechender Multiplexbetrieb ist auch für die Überwachungsschaltung 45 eingerichtet. Zur Sicherung gegen Programmausfälle kann ferner ein sog. Watchdog (nicht gezeigt) der Überwachungsschaltung 45 zugeordnet sein. Ein solcher Watchdog ist an sich bekannt und braucht hier nicht weiter erläutert zu werden.

Für die Stromversorgung der Meßelektronik 19 dienen wie erwähnt die vier wiederaufladbaren Batterien 18, bei denen es sich beispielsweise um vier NiCd-Akkus mit 500 mAh handelt. Diese Batterien 18 sind in Fig. 9 schematisch angedeutet, wobei ihre Anschlüsse 51 und 52 ebenso wie der zur eigentlichen Elektronik 19 führende Anschluß 53 zum oben erwähnten Steckverbindungsteil 17 geführt sind, um so entweder über die Anschlüsse 51, 52 die Akkus 18 nachladen zu können oder aber über eine Verbindungsbrücke am aufzusteckenden komplementären Steckerteil die Anschlüsse 52 und 53 miteinander zu verbinden und so die Meßelektronik 19 an die Spannungsquelle anzuschalten.

Der Meßelektronik 19 ist weiters, um eine Versorgung mit einem möglichst konstanten Strom zu gewährleisten, eine in Fig. 9 nur symbolisch dargestellte, an sich eine herkömmliche Bauart aufweisende Konstantstromquelle 54 zugeordnet. Diese Konstantstromquelle 54 sieht beispielsweise einen Konstantstrom von 1,298 mA vor, der in den jeweiligen Temperaturmeßwiderstand 9 bzw. 42 bzw. den Referenzwiderstand 43, je nach Stellung der Multiplexereinheit 44, fließt.

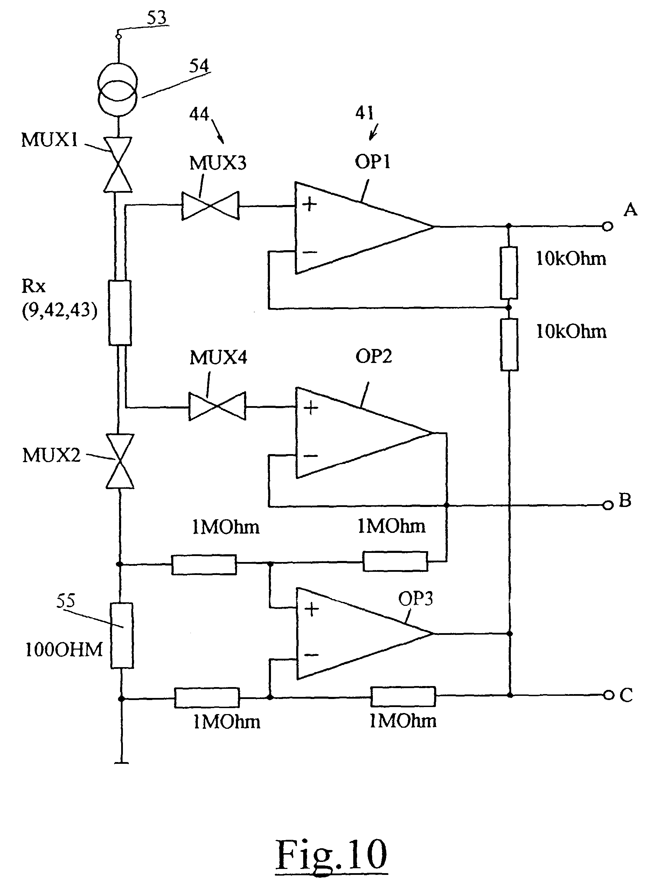

Wie mehr im Detail aus Fig. 10 ersichtlich ist, besteht die Multiplexereinheit 44 aus vier einzelnen Multiplexern MUX1, MUX 2, MUX3 und MUX4, wobei die Multiplexer MUX1 und MUX2 für die Stromflußmessung am jeweiligen Meßwiderstand 9 bzw. 42 bzw. 43, in Fig. 10 symbolisch mit $R_X$ bezeichnet (wobei es sich hierbei um jeweils einen der angegebenen acht Meß- bzw. Referenzwiderstände handelt), und die Multiplexer MUX3 und MUX4 für die Spannungsmessung zuständig sind. Diese Multiplexer MUX1 bis MUX4 sind beispielsweise durch die an sich herkömmlichen Multiplexerkomponenten vom Typ 4051 gebildet, wogegen es sich bei der Multiplexer-Überwachungsschaltung 45 beispielsweise um eine Schaltung vom Typ 4052 handelt.

Der in Fig. 9 ganz allgemein veranschaulichte und mit 41 bezeichnete analoge Meßsignalverstärker ist im einzelnen gemäß Fig. 10 mit drei Operationsverstärkern OP1, OP2 und OP3 aufgebaut, wobei die in Fig. 10 gezeigte Schaltung zugleich eine Meßschaltung zur Überprüfung der Gleichspannungsparameter Gain und Offset der Verstärkerschaltung 41 aufweist. In diesem Zusammenhang ist ein weiterer Präzisions-Referenzwiderstand 55 mit einem Wert von beispielsweise 100Ω vorgesehen, so daß an diesem Widerstand 55, wenn der Strom der Konstantstromquelle 54 die angegebenen 1,298 mA beträgt, eine Spannung von 129,8 mV abfällt. Demgegenüber beträgt im Normalfall die Spannung $U_{BC}$ zwischen den zwei Ausgangspunkten B und C 129,8 mV, und ganz allgemein ist die Spannung zwischen den Ausgangspunkten B und C ($U_{BC}$) ein Maß für den jeweiligen Gain-Wert der Verstärkerschaltung 41, d.h. der Referenzwiderstand 55 dient zur Erfassung etwaiger Gain-Änderungen, wobei diese Bestimmung unabhängig davon erfolgt, welcher Meßwiderstand $R_X$ (2 bzw. 42 bzw. 43) über die Multiplexer MUX1 bis MUX4 gerade an die Schaltung angeschaltet ist.

Der bereits erwähnte Referenzwiderstand 43 (s. Fig. 9) dient andererseits zur Bestimmung des Offset-Wertes der Analogverstärker-Schaltung 41, wobei dieser Referenzwiderstand 43 beispielsweise ebenfalls ein Präzisionswiderstand mit einem Wert von 100Ω ist. Der jeweilige Offset-Wert wird sodann aus der zwischen den Ausgangspunkten A und C liegenden Spannung $U_{AC}$ ermittelt; diese Spannung $U_{AC}$ errechnet sich dabei aus dem Spannungsabfall am Referenzwiderstand 43 (im vorgenannten Beispiel, mit einem Widerstandswert von 100Ω, also 129,8 mV), wobei von diesem Spannungsabfall die Spannung am Gain- Meßwiderstand 55 - mit Hilfe des Operationsverstärkers OP3 - abgezogen wird. Im Idealfall ergibt sich hier der Wert 0 V. Sofern sich jedoch der Widerstandswert des Referenzwiderstandes 43 ändert, etwa aufgrund von Temperaturerhöhungen, ergibt sich für die genannte Differenz ein bestimmter Wert, der durch die Verstärkerschaltung mit dem Operationsverstärker OP1 (der einen 2-fach - nicht invertierenden Operationsverstärker bildet, wogegen OP2 ein Spannungsfolger ist) verdoppelt wird. Es gilt also:

$$U_{AC} = 2 \cdot (R_X \cdot 1{,}298 \text{ mA} - 129{,}8 \text{ mV}).$$

Beispielsweise erhöht sich der Widerstandwert des Meßwiderstandes 9 (Fig. 9) bei einer Guttemperatur (Flaschentemperatur) von 100°C von 100Ω auf 138,5Ω, und demgemäß ergibt sich dann für $U_{AC}$ z.B. ein Wert von 99,24 mV.

Wie erwähnt wird dagegen die Verstärkung der Anordnung durch die Spannung $U_{BC}$ ständig überwacht und gemessen, und hier beträgt im Idealfall der Gainwert (die Verstärkung) 129,8.

Die Werte für diese Gleichspannungsparameter Offset und Gain werden wie angegeben über die in Fig. 9 schematisch veranschaulichte Verbindungsleitung 47 an die Multiplexer- Überwachungsschaltung 45 angelegt und von dieser über Signalleitungen 56 zur Digitalisierung dem A/D-Konverter 49 zugeführt.

Die beschriebene Verstärkungs- und Meßschaltung 41, 45 ergibt somit einen praktisch linearen Zusammenhang zwischen der jeweiligen Temperatur und der Ausgangsspannung der Meßschaltung, und die Schaltung kommt dabei ohne Einstellregler aus und verwendet im Meßteil nur Präzisionswiderstände mit z.B. 0,02% Toleranz bei 5 ppm°C. Die Schaltungskomponenten sind im weiteren auch so ausgelegt, daß die jeweilige Signalstärke (in mV) bereits ungefähr dem jeweiligen Meßwert (in °C) entspricht.

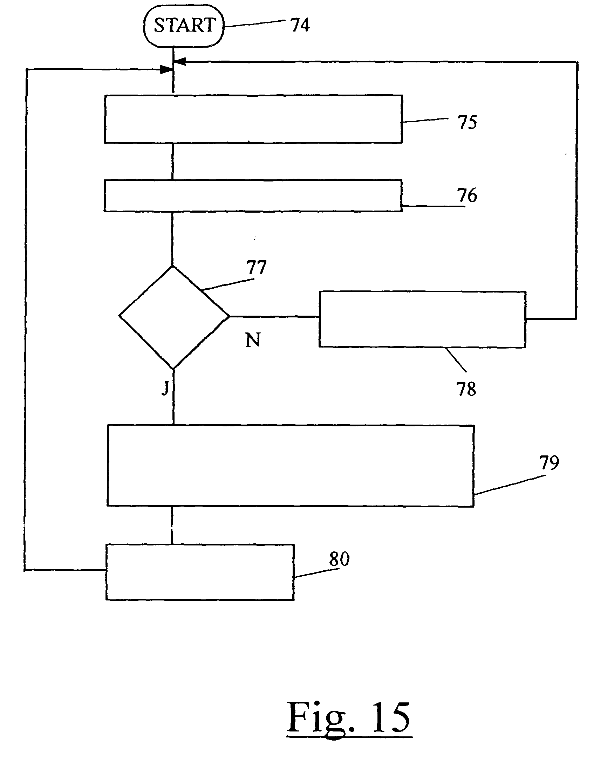

Die Werte für Offset und Gain werden in der Folge zusammen mit den Temperaturmeßwerten per Funk zur Empfängereinheit (Fig. 14) übermittelt, wo eine nachstehend noch näher - anhand der Fig. 15 - zu erläuternde Meßwertkorrektur vorgenommen wird.

Der Vollständigkeit halber sind in Fig. 9 weiters ein Controllbus 57 sowie - zwischen A/D-Konverter 49 und Mikrocontroller 50 - ein Datenbus 58 und ein Adressbus 59 veranschaulicht, wobei diese Bus-Leitungen in an sich herkömmlicher Weise vorgesehen sind. Weiters sei noch erwähnt, daß als A/D-Konverter 49 beispielsweise der Baustein Maxim 7109 Verwendung finden kann, und daß als Mikrocontroller 50 der Baustein 87C51 verwendet werden kann.

Der Mikrocontroller 50 steuert über den Controllbus 57 die verschiedenen Multiplexer und den A/D-Konverter 49, und er liefert Daten auf Basis der ihm über den Datenbus 58 gesandten Daten, entsprechend den Meßwerten der sechs Meßfühler 9 und des einen internen Meßfühlers 42 sowie entsprechend dem Offsetwert und dem Gainwert, und weiters entsprechend dem von der Überwachungsschaltung 45 über die Leitung 46 überwachten Akkuspannungswert (in %), über eine Leitung 60 an die Sendeeinheit 23, wobei der Mikrocontroller 50 diese Sendeeinheit 23 überdies abwechselnd ein- und ausschaltet. Dieses Ein- und Ausschalten kann abhängig von Änderungen in den Meßsignalen in kürzeren oder längeren Zeitabständen erfolgen, und bei Erreichen einer relativ stabilen Temperatursituation kann die Sendeeinheit 23 beispielsweise alle 3,5 s für eine Sendezeit von 0,6 s, zum Senden eines Datenpakets über die Sendeantenne 16, aktiviert werden.

Die Daten werden dabei vom Mikrocontroller 50 Hammingcodiert, so daß eine Fehlerkorrektur bzw. Fehlererkennung möglich ist. Im einzelnen wird durch die Hamming-Codierung die Korrektur von 1-Bit-Fehlern bei der Übertragung ermöglicht sowie die Erkennung von Mehrbit-Fehlern erlaubt.

Die Sendeeinheit 23 kann mit einer an sich herkömmlichen HF- Sendeschaltung, mit herkömmlichen Oszillator- und Filterkreisen, aufgebaut sein, wobei die Datenübertragung durch einfache Frequenzmodulation, nämlich insbesondere durch einfache Frequenzverschiebung der Sendefrequenz (Modulationsart F1) erfolgen kann.

In Fig. 11 ist ein Teil der Sendeeinheit 23 gezeigt, und zwar jener Schaltungsteil, der für die Frequenzverschiebung verantwortlich ist, wobei die nachfolgenden Kreise, wie Oszillator, Filter etc., wie erwähnt von herkömmlicher Bauart sind und daher nicht näher erläutert werden brauchen.

Im einzelnen werden die NF-Impulse (Datenimpulse) über die Leitung 60 an einen Anschluß 61 der Frequenverschiebungs-Schaltung 62 angelegt. In dieser Frequenzverschiebungs-Schaltung ist zusätzlich zu einem die HF-Frequenz bestimmenden Quarz 63 ein Kreis mit einem Abstimmkondensator (Trimmer) 64 sowie einer Kapazitätsdiode 65 aufgenommen. Mit dem Trimmer 64, der beispielsweise einen Bereich von 2 bis 10 pF hat, wird die Trägerfrequenz der Sendeeinheit 23 auf beispielsweise 27,255 MHz, auf 10 Hz genau, abgeglichen. Bei Anlegen von NF-Impulsen bewirkt die Kapazitätsdiode 65 sodann eine Verschiebung der Trägerfrequenz, wobei der Frequenzhub beispielsweise ± 1,5 kHz beträgt.

Das HF-Signal wird über die Sendeantenne 16 abgestrahlt, die beispielsweise ein Viertelwellenstrahler mit einer wirksamen Länge von 105 cm ist. Dabei wird beispielsweise als Antennendraht ein Edelstahldraht mit einem Durchmesser von 1,5 mm verwendet, und dieser Draht wird zu einer Helix (Wendel) mit einem Durchmesser von 15 mm und einer Länge von ca. 150 mm gewickelt. Über diese Helix wird ein Schrumpfschlauch augeschrumpft, der aus einem an sich herkömmlichen Kunststoffmaterial besteht, so daß insgesamt eine stabförmige Konfiguration erhalten wird, wie aus Fig. 12 ersichtlich ist, wobei dieser Stab am dem Steckerteil 37' gegenüberliegenden Ende mit einer Kappe 66 verschlossen wird. Die Sendeantenne 16 kann auch beispielsweise als Viertelwellenstrahler mit einer gestreckten Länge von 100 cm ausgeführt sein. Dadurch werden die Abstrahleigenschaften verbessert.

In Fig. 13 ist schematisch die zugehörige Empfangsantenne 11 veranschaulicht, wobei es sich hier um eine Rahmenantenne (Cubical-Quad-Antenne) handelt, die als kreisförmige Schleife im Inneren des Autoklav-Stators 2 angebracht ist. Im einzelnen besteht die Empfangsantenne 11 aus einem beispielsweise 2 mm starken Edelstahl-Runddraht, der in einem Abstand von ca. 5 cm von der Innenwand des Statorgehäuses des Autoklav-Stators 2 mit Hilfe von Abstandshaltern (nicht gezeigt) montiert ist. Die Empfangsantenne 11 ist ferner an zwei einander diametral gegenüberliegenden Punkten 67, 68 mit der Autoklaven-Innenwand verbunden, und das HF-Ausgangssignal wird an einer Stelle 69 in einem Abstand von beispielsweise 60 cm vom einen Wandanschlußpunkt 67 ausgekoppelt und über eine Koaxialleitung durch die Durchführung 10 zur eigentlichen Empfängereinheit (s. Fig. 14) weitergeleitet. Die wirksame Länge der Rahmenantenne 11 betägt dabei ungefähr 11 m, was der Umfangslänge von üblichen Kesselwänden von Rotationsautoklaven entspricht (Durchmesser ungefähr 2,5 m), wodurch sich in der Folge auch die oben angegebene HF-Sendefrequenz von ungefähr 27 MHz ergibt. Mit einer Empfangsantenne 11 in Form einer solchen Rahmenantenne wie beschrieben wird auch bei umlaufendem Autoklav-Rotor 3 immer ein störungsfreier Empfang der von der Sendeeinheit 23 über die Antenne 16 gesendeten HF-Signale, trotz der herrschenden schwierigen Bedingungen, wie Wasserdampf, Abschirmung durch den Rotorkäfig etc., sichergestellt, wie dies auch Versuche erwiesen haben.

In Fig. 14 ist ein Blockschaltbild der gesamten Empfängereinheit 70 samt Empfangsantenne 11 veranschaulicht. Die Empfängereinheit 70 weist dabei eine an die Antenne 11 angeschlossene HF-Empfängerschaltung 71 auf, bei der es sich beispielsweise um einen völlig herkömmlichen Überlagerungsempfänger handeln kann. Das demodulierte NF-Ausgangssignal dieser Empfängerschaltung 71 gelangt sodann an eine Impulsformerstufe 72, und das so wiedergewonnene digitale Datensignal wird dann einem Mikrocontrollersystem 73 zugeführt. Der in diesem Mikrocontrollersystem 73 zusammen mit anderen Bausteinen, wie Arbeitsspeicher (RAM) und Programmspeicher (EPROM), in an sich herkömmlicher Weise zusammengefaßte Mikrocontroller decodiert das zugeführte digitale Signal (Hamming-Code) und führt eine Überprüfung auf Plausibilität durch. In diesem Zusammenhang wird auch auf das Flußdiagramm gemäß Fig. 15 verwiesen, wo der Programmablauf des Mikrokontrollersystems 73 veranschaulicht ist.

Im einzelnen wird gemäß Fig. 15 nach einem Startschritt bei 74 in einem Schritt 75 das jeweilige Datenpaket empfangen und decodiert. In einem anschließenden Schritt 76 wird die Hamming-Decodierung und der Plausibilitätscheck durchgeführt, wobei die entsprechende Abfrage, ob das Datenpaket akzeptabel ist oder nicht, bei 77 erfolgt. Bei der Plausibilitätsüberprüfung kann beispielsweise überprüft werden, ob die Temperaturänderung von einem Meßzyklus zum anderen kleiner als ± 5°C ist, wobei bei stärkeren Änderungen eine fehlerhafte Messung angenommen wird. Ist das Ergebnis der Abfrage bei 77 negativ, so werden gemäß Block 78 die Daten als ungültig angenommen, und es wird wieder zum Block 75 zurückgekehrt, um das nächste Datenpaket zu empfangen und zu decodieren.

Ist dagegen das Ergebnis der Überprüfung gemäß Block 77 positiv, d.h. die Daten werden als plausibel erkannt, so wird beim Block 79 eine Meßwertkorrektur gemäß folgender Beziehung durchgeführt:

$$T(ist) = T(gemessen) . Gain/129,8 + Offset$$

Es werden somit hier Änderungen der Gleichspannungsparameter des analogen Meßsignalverstärkers 41 (s. Fig. 9) bei der Meßwertbestimmung berücksichtigt, wobei sich der Gain-Bezugswert von 129,8 im vorliegenden Beispiel aus dem oben angegebenen Wert für den Konstantstrom der Konstantstromquelle 54 ergibt.

In einem abschließenden Schritt 80 werden sodann die decodierten, überprüften und korrigierten Daten an einen externen Rechner geschickt, beispielsweise über eine Schnittstelle RS232/V24, wie auch in Fig. 14 schematisch bei 81 veranschaulicht ist. Danach wird im Programm wieder zum Beginn zurückgekehrt.

Bei dem in der Zeichnung nicht näher veranschaulichten externen Rechner kann es sich insbesondere um einen Personal Computer (PC) handeln, und in diesem Rechner kann auch eine weitere Bearbeitung der Temperaturdaten, nämlich insbesondere eine Kennlinienkalibrierung, vorgenommen werden; diese Kennlinienkalibrierung erfolgt dabei auf Basis von vorhergehend vorgenommenen Eichungen der Temperatur-Meßfühler 9 für einzelne Teilbereiche des gesamten Meßbereiches, um so zu einer hohen Gesamt-Meßgenauigkeit zu kommen. Dabei kann, wie Versuche gezeigt haben, eine Genauigkeit der Messung von ± 0,15°C über den gesamten Meßbereich (z.B. von 0 bis 150°C) erzielt werden. Hierdurch ist eine außerordentlich genaue Prozeßüberwachung bei der Sterilisation von pharmazeutischen Produkten möglich, was von besonderer Bedeutung ist, da dort vorgegebene Temperaturen mit großer Genauigkeit über bestimmte Zeiträume eingehalten werden müssen. Beispielsweise beträgt die einzuhaltende Sterilisationstemperatur bei einer Sterilisation mit Wasserdampf 121°C, mit einer Sterilisationszeit von 20 min. Relativ geringfügige Temperaturabweichungen könnten dabei bereits zur Notwendigkeit der Wiederholung der Sterilisationsbehandlung führen, jedoch wird im vorliegenden Fall eine On-Line-Regelung ermöglicht, so daß bei beginnenden Änderungen der Sterilisationstemperatur sofort eingegriffen und die Temperatur wieder auf den gewünschten Wert geregelt werden kann. Dadurch gelingt es, insgesamt relativ kurze und doch verläßliche Sterilisationsbehandlungen sicherzustellen, so daß mit der vorliegenden Anordnung insgesamt eine erhebliche Zeit- und Kostenersparnis verbunden ist.

Wenn die Erfindung vorstehend anhand von besonders bevorzugten Ausführungsbeispielen näher erläutert wurde, so sind doch selbstverständlich Abwandlungen und Modifikationen im Rahmen der Ansprüche möglich. So können z.B. anstatt der Temperaturmeßwiderstände (Pt 100) als Meßfühler auch andere, an sich bekannte Fühlerbausteine, wie etwa Halbleitermeßfühler etc., verwendet werden. Ferner kann zur Energieversorgung der Meßelektronik in der Meßflasche eine induktive Energieübertragung vorgenommen werden, wobei eine Spule z.B. den Autoklav-Rotor umschließt und mit der Meßelektronik in der Meßflasche verbunden ist, und wobei eine zugehörige weitere Spule (Primärwicklung) im Inneren des Autoklav-Stators angebracht ist.

**Patentansprüche**

1. Meßanordnung für Einrichtungen mit erschwerten klimatischen Bedingungen, z.B. Autoklaven (1), mit mindestens einem Meßfühler (9) zur Erfassung von physikalischen Meßgrößen, z.B. einem Temperaturmeßfühler, mit einer mit dem Meßfühler (9) über eine Verbindungsleitung (26) verbundenen bzw. verbindbaren Meßelektronik (19), die in einem thermisch isolierten Behälter (8) angeordnet ist und eine Sendeeinheit (23) enthält, der eine außerhalb des Behälters (8) an einem Deckel (13) hiefür angeordnete, über eine Verbindungsleitung angeschlossene bzw. anschließbare Sendeantenne (16) zugeordnet ist, und mit einer gesonderten Empfängereinheit (70), der eine Empfangsantenne (11) zugeordnet ist, dadurch gekennzeichnet, daß der thermisch isolierte Behälter (8) zwei zueinander entgegengesetzt ausgerichtete, ineinander angeordnete Dewar-Gefäße (20, 21) enthält, von denen das innere Dewar-Gefäß (21) die Meßelektronik (19) aufnimmt, wobei die Verbindungsleitungen (26) im Zwischenraum zwischen den Dewar- Gefäßen (20, 21) hindurchgeführt und an im Deckel (13) dicht angebrachten Kupplungsteilen (14, 15) angeschlossen sind, denen korrespondierende Kupplungsteile (37) zum Anschließen der Sendeantenne (16) bzw. der Verbindungsleitung zum wenigstens einen Meßfühler (9) zugeordnet sind, und daß die Meßelektronik (19) einen Analog/Digital-Konverter (49) zur Digitalisierung der Meßdaten sowie einen digitalen Steuer- und Prozessorbaustein (50) enthält, dem die Sendeeinheit (23) nachgeschaltet ist.

2. Meßanordnung nach Anspruch 1, dadurch gekennzeichnet, daß im Deckel (13) des thermisch isolierten Behälters (8) weiters ein mit einer aufladbaren Batterie (18) bzw. mit Stromversorgungsklemmen der Meßelektronik (19) verbundener Kupplungsteil (17) angebracht ist.

3. Meßanordnung nach Anspruch 1 oder 2, dadurch gekennzeichnet, daß die Kupplungsteile (14, 15, 17) Steckverbindungsteile sind.

4. Meßanordnung nach einem der Ansprüche 1 bis 3, dadurch gekennzeichnet, daß die beiden Dewar-Gefäße (20, 21) in einer Isolierschicht (22) aus Aluminiumoxidkeramik eingebettet sind.

5. Meßanordnung nach einem der Ansprüche 1 bis 4, dadurch gekennzeichnet, daß der Prozessor- und Steuerbaustein (50) einen Codierer zur Codierung der digitalisierten Meßdaten, z.B. einen Hamming-Codierer, enthält.

6. Meßanordnung nach einem der Ansprüche 1 bis 5, dadurch gekennzeichnet, daß der Prozessor- und Steuerbaustein (50) die Sendeeinheit (23) abwechselnd ein- und ausschaltet.

7. Meßanordnung nach einem der Ansprüche 1 bis 6, dadurch gekennzeichnet, daß zur Überwachung der Temperatur im Inneren der Dewar-Gefäße (20, 21), im Bereich der Meßelektronik (19), ein interner Temperaturmeßfühler (42) angeordnet ist.

8. Meßanordnung nach einem der Ansprüche 1 bis 7, dadurch gekennzeichnet, daß im Fall von mehreren Meßfühlern (9, 42) diesen in an sich bekannter Weise eine Multiplexereinheit (44) für einen Multiplex-Betrieb der Meßfühler, zugeordnet ist.

9. Meßanordnung nach einem der Ansprüche 1 bis 8, dadurch gekennzeichnet, daß die Meßelektronik (19) eine dem Analog/Digital-Konverter (49) vorgeschaltete Überwachungsschaltung (45) für Gleichspannungsparameter, wie insbesondere Gain und Offset, eines eingangsseitigen, analogen Meßsignal-Verstärkers (41) der Meßelektronik (19) enthält, und von der Überwachungsschaltung (45) abgegebene, auf diese Gleichspannungsparameter bezogene Überwachungssignale nach Digitalisierung im Analog/Digital-Konverter (49) an den Prozessor- und Steuer-Baustein (50) zur Übermittlung an die Empfängereinheit (70) angelegt werden.

10. Meßanordnung nach Anspruch 9, dadurch gekennzeichnet, daß die Überwachungsschaltung (45) mit einer Meßschaltung mit Referenzwiderständen (43, 55) ausgebildet ist, wobei im Betrieb der jeweilige Spannungsabfall am Meßwiderstand (9) zwecks Bestimmung von Gain bzw. Offset gemessen wird.

11. Meßanordnung nach einem der Ansprüche 1 bis 10, dadurch gekennzeichnet, daß der Prozessor- und Steuerbaustein (50) mit einer einen Eingangsteil der Sendeeinheit (23) bildenden Frequenzverschiebungsschaltung (62) verbunden ist, die bei Anlegen von Datenimpulsen durch den Prozessor- und Steuerbaustein (50) eine Verschiebung der Sendefrequenz der Sendeeinheit (23) bewirkt.

12. Meßanordnung nach einem der Ansprüche 1 bis 11, dadurch gekennzeichnet, daß die Empfangsantenne (11) durch einen zur Anbringung längs der Innenfläche einer äußeren Autoklaven- Wand vorgesehene, eine geschlossene, allgemein kreisförmige Schleife bildende Rahmenantenne gebildet ist.

13. Meßanordnung nach einem der Ansprüche 1 bis 12, dadurch gekennzeichnet, daß in der Empfängereinheit (70) ein Digitalisierungsbaustein (72) sowie ein Prozessorsystem (73) gegebenenfalls mit einem dem Codierer des Prozessor- und Steuerbausteins (50) der Meßelektronik (19) im thermisch isolierten Behälter (8) entsprechenden Decoder, vorgesehen ist, um die aktuellen Meßdaten, wie Fühlertemperaturen, aus den durch Funk übertragenen Signalen zu gewinnen.

14. Meßanordnung nach Anspruch 13, gekennzeichnet durch eine Verbindung (81) des Prozessorsystems (73) zu einem externen Rechner, um diesem die Meßdaten für eine On-line-Auswertung zu übermitteln.

**Claims**

1. A measuring arrangement for devices under heavy climatic conditions, e.g. autoclaves (1), comprising at least one measuring probe (9) for detecting physical quantities to be measured, e.g. a temperature measuring probe, a measuring electronics (19) connected, or adapted to be connected, respectively, with the measuring probe (9) via a connection line (26) and arranged in a thermally insulated container (8), which measuring electronics includes a transmitter unit (23) to which a transmitting antenna (16) is associated which is arranged outside of the container (8) on a lid (13) therefor and is connected, or adapted to be connected, respectively, via a connection line, and a separate receiver unit (70) to which a receiver antenna (11) is associated, characterised in that the thermally insulated container (8) comprises two oppositely oriented Dewar vessels (20, 21) arranged one inside the other, the inner Dewar vessel (21) accommodating the measuring electronics (19), wherein the connection lines (26) are led through the interspace between the Dewar vessels (20, 21) and are connected to coupling elements (14, 15) tightly provided in the lid (13), to which corresponding coupling elements (37) are associated for connecting the transmitting antenna (16), and the connection line, respectively, to the at least one measuring probe (9), and that the measuring electronics (19) comprises an analog/digital converter (49) for digitalizing the measured data as well as a digital control and processor module (50) followed by the transmitter unit (23).

2. A measuring arrangement according to claim 1, characterised in that furthermore a coupling element (17) connected to a rechargeable battery (18), or to power supply terminals of the measuring electronics (19), respectively, is attached to the lid (13) of the thermally insulated container (8).

3. A measuring arrangement according to claim 1 or 2, characterised in that the coupling elements (14, 15, 17) are plug connection elements.

4. A measuring arrangement according to any one of claims 1 to 3, characterised in that the two Dewar vessels (20, 21) are embedded in an insulation layer (22) of aluminum oxide ceramics.

5. A measuring arrangement according to any one of claims 1 to 4, characterised in that the processor and control module (50) comprises a coding means, e.g. a Hamming coder, for coding the digitalized measured data.

6. A measuring arrangement according to any one of claims 1 to 5, characterised in that the processor and control module (50) alternately switches on and off the transmitter unit (23).

7. A measuring arrangement according to any one of claims 1 to 6, characterised in that an internal temperature measuring probe (42) is arranged in the region of the measuring electronics (19) to monitor the temperature in the interior of the Dewar vessels (20, 21).

8. A measuring arrangement according to any one of claims 1 to 7, characterised in that, if several measuring probes (9, 42) are provided, a multiplexer unit (44) for multiplex operation of the measuring probes is associated to the same in a manner known per se.

9. A measuring arrangement according to any one of claims 1 to 8, characterised in that the measuring electronics (19) comprises a monitoring circuit (45) for DC parameters, such as, in particular, gain and offset, of an input-side analog measuring signal amplifier (41) of the measuring electronics (19), which monitoring circuit precedes the analog/digital converter (49), and that monitoring signals delivered by the monitoring circuit (45) and related to these DC parameters are applied to the processor and control module (50) after digitalization in the analog/digital converter (49) for transmission to the receiver unit (70).

10. A measuring arrangement according to claim 9, characterised in that the monitoring circuit (45) is designed to comprise a measuring circuit including reference resistors (43, 55), wherein in operation the respective voltage drop at the measuring resistor (9) is measured to determine gain and offset, respectively.

11. A measuring arrangement according to any one of claims 1 to 10, characterised in that the processor and control module (50) is connected to a frequency shift circuit (62) forming an input component of the transmitter unit (23), which causes a shift in the transmission frequency of the transmission unit (23) at the application of data pulses by the processor and control module (50).

12. A measuring arrangement according to any one of claims 1 to 11, characterised in that the receiving antenna (11) is formed by a frame antenna to be attached to an external autoclave wall along the internal surface formed and forming a closed, generally circular loop.

13. A measuring arrangement according to any one of claims 1 to 12, characterised in that a digitalization module (72) as well as a processor system (73), optionally together with a decoder corresponding to the coder of the processor and control module (50) of the measuring electronics (19) placed within the thermally insulated container (8), are provided in the receiver unit (70) to gain the current measured data, such as probe temperatures, from the radio-transmitted signals.

14. A measuring arrangement according to claim 13, characterised by a connection (81) of the processor system (73) to an external computer to transmit the measured data to the same for an on-line evaluation.

**Revendications**

1. Agencement de mesure pour dispositifs à conditions climatiques sévères, par exemple des autoclaves (1), comprenant au moins un capteur de mesure (9) pour la saisie de grandeurs de mesure physique, par exemple un capteur de mesure de la température, une électronique de mesure (19) reliée ou pouvant être reliée au capteur de mesure (9) par l'intermédiaire d'une ligne de connexion (26), l'électronique de mesure étant agencée dans un récipient (8) thermiquement isolé et contenant une unité d'émission (23) à qui est associée une antenne d'émission (16) agencée à cet effet à l'extérieur du récipient (8) sur un couvercle (13) et connecté ou pouvant être connecté par l'intermédiaire d'une ligne de connexion, et une unité de réception (70) particulière à qui est associée une antenne de réception (11), caractérisé en ce que le récipient (8) thermiquement isolé contient deux vases Dewar (20, 21) orientés dans des sens opposés l'un par rapport à l'autre, disposés l'un dans l'autre, dont le vase Dewar (21) intérieur reçoit l'électronique de mesure (19), les lignes de connexion (26) étant guidées dans l'espace intermédiaire entre les vases Dewar (20, 21) et connectées à des pièces d'accouplement (14, 15) fixées de façon étanche au couvercle (13) et à qui sont associées des pièces d'accouplement (37) correspondantes pour le raccordement de l'antenne d'émission (16) ou de la ligne de connexion à au moins un capteur de mesure (9), et en ce que l'électronique de mesure (19) comprend un convertisseur analogique-numérique (49) pour la numérisation des données de mesure, ainsi qu'une unité de processeur et de commande (50) numérique à laquelle est connectée à la suite l'unité d'émission (23).

2. Agencement de mesure selon la revendication 1, caractérisé en ce que, de plus, dans le couvercle (13) du récipient (8) thermiquement isolé, une pièce d'accouplement (17) reliée à une pile (18) rechargeable ou à des bornes d'alimentation de courant, est agencée.

3. Agencement de mesure selon la revendication 1 ou 2, caractérisé en ce que les pièces d'accouplement (14, 15, 17) sont des pièces de connexion à fiches.

4. Agencement de mesure selon l'une quelconque des revendications 1 à 3, caractérisé en ce que les deux vases Dewar (20, 21) sont enrobés d'une couche isolante (22) réalisée en céramique d'oxyde d'aluminium.

5. Agencement de mesure selon l'une quelconque des revendications 1 à 4, caractérisé en ce que l'unité de processeur et de commande (50) comporte un codeur pour le codage des données de mesure numérisées, par exemple en un codeur de Hamming.

6. Agencement de mesure selon l'une quelconque des revendications 1 à 5, caractérisé en ce que l'unité de processeur et de commande (50) branche et débranche alternativement l'unité d'émission (23).

7. Agencement de mesure selon l'une quelconque des revendications 1 à 6, caractérisé en ce qu'un capteur de mesure de la température (42) interne est agencé dans la zone de l'électronique de mesure (19) pour la surveillance de la température à l'intérieur des vases Dewar (20, 21).

8. Agencement de mesure selon l'une quelconque des revendications 1 à 7, caractérisé en ce que, dans le cas de plusieurs capteurs de mesure (9, 42), une unité de multiplexage (44) pour un fonctionnement multiplex des capteurs de mesure est associé de façon connue en soi à ces capteurs de mesure.

9. Agencement de mesure selon l'une quelconque des revendications 1 à 8, caractérise en ce que l'électronique de mesure (19) comporte un circuit de surveillance (45) connecté en amont du convertisseur analogique numérique (49), pour des paramètres de tension continue, comme notamment le gain et l'offset, d'un amplificateur (41) analogue du côté entrée, du signal de mesure de l'électronique de mesure (19) et en ce que des signaux de surveillance émis par le circuit de surveillance (45), se rapportant à ces paramètres de tension continue, sont mis à disposition après la numérisation dans le convertisseur analogique numérique (49), à l'unité de processeur et de commande (50) pour la transmission à l'unité de réception (70).

10. Agencement de mesure selon la revendication 9, caractérisé en ce que le circuit de surveillance (45) comprend un circuit de mesure avec des résistances de référence (43, 55), en fonctionnement, la tension correspondante de la résistance de mesure (9) étant mesurée pour la détermination du gain ou de l'offset.

11. Agencement de mesure selon l'une quelconque des revendications 1 à 10, caractérisé en ce que l'unité de processeur et de commande (50) est connectée à un circuit de décalage de fréquence (62) formant la partie d'entrée de l'unité d'émission (23), le circuit de décalage de fréquence provoquant un décalage de la fréquence d'émission de l'unité d'émission (23) lors de la mise à disposition d'impulsions de données par l'unité de processeur et de commande (50).

12. Agencement de mesure selon l'une quelconque des revendications 1 à 11, caractérisé en ce que l'antenne de réception (11) est formée par une antenne cadre formant une boucle de forme générale circulaire, fermée prévue le long de la surface intérieure d'une paroi extérieure d'autoclave pour le montage.

13. Agencement de mesure selon l'une quelconque des revendications 1 à 12, caractérisé en ce que dans l'unité de réception (70), une unité de numérisation (72) ainsi qu'un système de processeur (73) comprenant le cas échéant un décodeur correspondant au codeur de l'unité de processeur et de commande (50) de l'électronique de mesure (19) dans le récipient (8) thermiquement isolé, est prévue pour obtenir à partir des signaux transmis par radio des données de mesure actuelles, comme des températures du capteur.

14. Agencement de mesure selon la revendication 13, caractérisé par une liaison (81) du système de processeur (73) à un calculateur externe, pour transmettre des données de mesure pour une exploitation en direct à ce calculateur.

Fig.2

Fig.1

# Fig.3

Fig.5

Fig.4

<u>Fig.6</u>

<u>Fig.7</u>

<u>Fig.8</u>

Fig.9

# Fig.10

## Fig.11

62

64

65

(von 60) 61

63

## Fig.12

66

16

39      37'

Fig.13

70

11

71

72

73

81

Fig.14

START — 74

75

76

77

N

78

J

79

80

Fig. 15